# EUROPEAN PATENT APPLICATION

(11) **EP 2 221 044 A1**
(43) Date of publication of application: **25.08.2010**
(21) Application number: 09153412.3
(22) Date of filing: 23.02.2009
(51) Int. Cl.: A61K 8/81, A61K 8/73, A61Q 5/06

(54) **Cosmetic composition comprising a cationic polymer and chitosan**

(71) Applicant: The Procter and Gamble Company, Cincinnati, Ohio 45202 (US)
(72) Inventor: Loifenfeld, Marina, 60316, Frankfurt (DE); Birkel, Susanne, 64295, Darmstadt (DE)
(74) Representative: Hirsch, Uwe Thomas M.H.

(57) **Abstract**

A cosmetic composition comprising a first polymer being a cationic polymer, a second polymer selected from chitosan, salts and derivatives thereof, and a cosmetically acceptable carrier. This composition is suitable for achieving long-lasting hold, high hair-setting properties and exhibiting satisfactory consistency/rheology altogether with exhibiting appealing characteristics.

## Description

### FIELD OF THE INVENTION

According to a first aspect, the present invention relates to a cosmetic composition comprising a first polymer being a cationic polymer, a second polymer being chitosan, chitosan salts, chitosan derivatives or mixtures thereof, and a cosmetically acceptable carrier. According to further aspects, it also relates to a method of treating hair, and the use of this cosmetic composition for achieving an effect on hair.

### BACKGROUND OF THE INVENTION

Amongst cosmetic compositions, hair styling compositions are intended to be used for achieving individual hair styles, and for temporarily holding hairs in place for a period of time.

These compositions may be provided in various forms such as mousses, gels and waxes. Particularly, hair styling compositions in the form of a gel are popular with consumers and users. These compositions are easy to apply onto hair, e.g. as a hand gel or as a spray gel, and they have appealing characteristics, particularly when in transparent or translucent form.

In addition to providing hold and setting to hair, hair styling compositions may impart other benefits, such as hair volume, hair curl definition, reduced hair frizz, hair shine effects, and particular aesthetic appearances such as a wet look or a crispy appearance.

Hair styling compositions usually comprise at least one hair fixing polymer. Many hair fixing polymers are available, including anionic, amphoteric, non-ionic and/or cationic hair fixing polymers. Incorporating hair fixing polymers, particularly cationic hair fixing polymers, enables the provision of a composition that can impart a degree of setting, e.g. from natural to ultrastrong hold, and various hold factors, e.g. from short-term to long-term (more than 24 hours) hold to hair style.

Hair styling compositions may also comprise at least one thickening polymer, for example in compositions in the form of a gel, in order to impart a degree of viscosity or firmness. Anionic thickening polymers are commonly used to obtain compositions with a satisfactory rheology/consistency. However, anionic thickening polymers may impair the properties of the composition when formulated with certain hair fixing polymers, particularly cationic hair fixing polymers. In fact, hair styling compositions comprising both an anionic thickening polymer and a cationic hair fixing polymer exhibit, over time, a lumpy consistency, typically with aesthically unpleasant, white crumbly lumps. In addition, after applying such compositions onto hair, residues may persist on the hair and this may alter the aesthetic appearance of hair, and/or impart a sticky, rough or unpleasant sensory feel to hair and skin.

There is a constant need, therefore, for providing a cosmetic composition, preferably a hair styling composition, to be used for achieving individual hair styles having long-lasting hold with high hair-setting properties. Particularly important for achieving a long-lasting hold is the humidity resistance properties of a hair styling composition. There is also a need for providing a cosmetic composition exhibiting a satisfactory consistency/rheology. Particularly, there is a need for providing a cosmetic composition achieving long-lasting hold, high hair-setting properties and exhibiting satisfactory consistency/rheology combined with appealing characteristics.

In addition, the composition is applied onto and/or distributed over hair with the hands, hence there is a need for the composition to have good sensory feel, good hair-setting effects and good aesthetic characteristics. It is preferable the composition to feel smooth rather than rough in the hand and to be easily distributed across the skin of the hands.

Furthermore, there is a need for providing a cosmetic composition leaving minimum residues onto hair after application and not imparting sticky, rough or unpleasant sensory feel to hair and skin.

Finally, there is also a constant need for providing hair styling compositions also achieving increased hair volume, reduced frizziness of the hair, improved hair curl definition, and improved hair shine effects. Particularly, there is a need for providing hair styling compositions achieving a wet look or a crispy appearance to the hairstyle.

### SUMMARY OF THE INVENTION

According to a first aspect, the present invention relates to a cosmetic composition comprising:
(a) a first polymer comprising at least one amino-substituted vinyl unit, at least one hydrophobic non-ionic vinyl unit, at least one associative vinyl unit, and at least one semi-hydrophobic vinyl surfactant unit;
(b) a second polymer selected from chitosan, chitosan salts, chitosan derivatives or mixtures thereof;
(c) a cosmetically acceptable carrier.

According to a second aspect, the present invention relates to a method of treating hair comprising the steps of:
(a) providing a composition, according to the first aspect of the invention,
(b) applying said composition onto hair and/or the scalp; and,
(c) achieving a hairstyle.

According to a third aspect, the present invention relates to a use of a composition, according to the first aspect of the invention, for achieving a hair style having at least one of curl definition, shine, hold and/or volume.

As used herein, the term "gel" means a composition having a viscosity from 20000 to 40000 mPa·s measured at 25°C using the device Brookfield DV-III Ultra Programmable Rheometer from Brookfield Engineering Laboratories.

As used herein, the term "long-lasting hold" means the hold factor conferred by the hair style over a period of time and/or under determined conditions of humidity, by applying a hair styling composition onto hairs.

As used herein, the terms "weight percentage" and "percentage by weight" of a component mean the percentage of active component and not the percentage of the raw material comprising this active, unless otherwise specified (see examples).

All percentages, ratios and proportions herein are by weight, unless otherwise specified.

### DETAILED DESCRIPTION OF THE INVENTION

According to the first aspect, the present invention relates to a cosmetic composition comprising a first polymer being a cationic polymer, a second polymer being selected from chitosan, salts, derivatives or mixtures thereof, and a cosmetically acceptable carrier.

The inventors have surprisingly found that the incorporation of a specific cationic thickening polymer - as described hereafter - and a second polymer selected from chitosan, chitosan salts, chitosan derivatives and/or mixtures enables the obtainment of a cosmetic composition, e.g. a hair styling composition, achieving long-lasting hold and high hair-setting properties, and exhibiting satisfactory consistency/rheology combined appealing characteristics. Particularly, the inventors have found that incorporating such polymers enables the obtainment of compositions that leave a minimum of - if any - residues onto hair and not imparting a sticky, rough or unpleasant sensory feel to hair and skin. More generally, the inventors have found that such composition achieves a hair style with superior curl definition, shine, hold and volume, impairing neither the application experience nor the aesthetics of the composition.

The composition comprises a first polymer being a cationic polymer. This polymer has been found to impart desirable rheological properties to hair styling compositions. These polymers are generally basic, aqueous, acid-swellable, or aqueous acid-soluble, polymers and salts thereof, which contain at least one basic amino substituent that is cationic at low pH, at least one hydrophobically modified polyoxyalkylene substituent derived from an associative vinyl monomer, and at least one polyoxyalkylene substituent derived from a semi-hydrophobic vinyl surfactant monomer.

This first polymer comprises at least one amino-substituted vinyl unit, at least one hydrophobic non-ionic vinyl unit, at least one associative vinyl unit, and at least one semi-hydrophobic vinyl surfactant unit.

The amino-substituted vinyl unit is selected from monomers being a mono-(C₁-C₄)alkylamino(C₁-C₈)alkyl (meth)acrylate; di-(C₁-C₄)alkylamino(C₁-C₈)alkyl (meth)acrylate; mono-(C₁-C₄)alkylamino(C₁-C₈)alkyl (meth)acrylamide; di-(C₁-C₄)alkylamino(C₁-C₈)alkyl (meth)acrylamide; nitrogen-containing heterocyclic (meth)acrylamide; nitrogen-containing heterocyclic (meth)acrylate; or mixtures thereof. Preferably, the amino-substituted vinyl unit is selected from 3-(N,N-dimethylamino)propyl (meth)acrylate, and N'- (3-N, N-dimethylamino) propyl (meth) acrylamide, or mixtures thereof. Most particularly preferred are 2-(N,N-dimethylamino)ethyl methacrylate, 2-(N,N-diethylamino)ethyl methacrylate, 2-(tert-butylamino)ethyl methacrylate, 2-(N,N-dimethylamino)propyl methacrylamide, and 2-(N,N-dimethylamino)neopentyl acrylate, or mixtures thereof.

Preferably, the first polymer comprises from about 20 to about 50 weight percent, which is of the total monomer mixture weight prior to polymerisation of the first polymer, of at least one amino-substituted vinyl monomer.

The hydrophobic non-ionic vinyl unit is selected from monomers being a C₁-C₃₀ alkyl (meth)acrylate; C₁-C₃₀ alkyl (meth)acrylamide; styrene; substituted styrene; vinyl ester; unsaturated nitrile; unsaturated silane; or mixtures thereof. Preferably, the hydrophobic non-ionic vinyl unit is selected from a C₁-C₃₀ alkyl ester of acrylic acid, C₁-C₃₀ alkyl ester of methacrylic acid, or a mixture thereof.

Preferably, the first polymer comprises from about 50 to about 65 weight percent, which is of the total monomer mixture weight prior to polymerisation of the first polymer, of at least one hydrophobic non-ionic vinyl monomer.

The associative vinyl unit is selected from monomers having the formula (I): 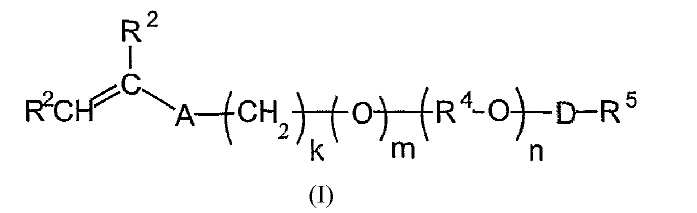 wherein R² is independently H, methyl, -C(O)OH, or -C(O)OR³; wherein R³ is a C₁-C₃₀ alkyl; A is -CH₂C(O)O-, -C(O)O-, -O-, -CH₂O-, -NHC(O)NH-, -C(O)NH-, -aryl-(CE₂)₂-NHC(O)O-, - aryl-(CE₂)₂-NHC(O)NH-, or -CH₂CH₂NHC(O)-; wherein aryl is a divalent aryl; E is hydrogen or methyl; z is 0 or 1; k is an integer in the range of 0 to 30, m is an integer being 0 or 1, with the proviso that when k is 0, m is 0, and when k is in the range of 1 to about 30, m is 1; (R⁴-O)ₙ is a polyoxyalkylene, which is a homopolymer, a random copolymer, or a block copolymer of C₂-C₄ oxyalkylene units, wherein R⁴ is C₂H₄, C₃H₆, C₄H₈, or a mixture thereof, and n is an integer in the range of 5 to 250, preferably 5 to 100, more preferably 10 to 80, and most preferably 15 to 60; D is a -R₄O-, -R⁴NH-, -C(O)-, -C(O)NH-, -R⁴NHC(O)NH-, or -C(O)NHC(O)-; and R⁵ is a substituted or unsubstituted alkyl selected from the group consisting of a C₈-C₄₀ linear alkyl, C₈-C₄₀ branched alkyl, C₈-C₄₀ carbocyclic alkyl, C₂-C₄₀ alkyl-substituted phenyl, aryl-substituted C₂-C₄₀ alkyl, and C₈-C₈₀ complex ester; wherein the R⁵ alkyl group optionally comprises one or more substituents selected from the group consisting of a hydroxyl group, alkoxyl group, and a halogen group. Preferably, the associative vinyl unit is selected from a cetyl polyethoxylated methacrylate, cetearyl polyethoxylated methacrylate, stearyl polyethoxylated (meth)acrylate, arachidyl polyethoxylated (meth)acrylate, behenyl polyethoxylated methacrylate, lauryl polyethoxylated methacrylate, cerotyl polyethoxylated (meth)acrylate, monthanyl polyethoxylated (meth)acrylate, melissyl polyethoxylated (meth)acrylate, lacceryl polyethoxylated (meth)acrylate, tristyryl phenolpolyethoxylated methacrylate, hydrogenated castor oil polyethoxylated methacrylate, canola polyethoxylated (meth)acrylate, cholesterol polyethoxylated methacrylate, and mixtures thereof.

Preferably, the first polymer comprises from about 0.1 to about 10 weight percent, which is of the total monomer mixture weight prior to polymerisation of the first polymer, of at least one associative vinyl monomer.

The semi-hydrophobic surfactant unit has the formula (II): 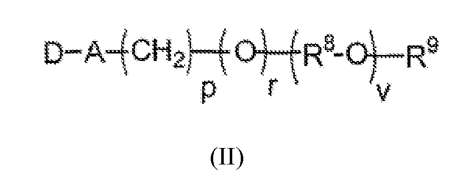 wherein A is -CH₂C(O)O-, -C(O)O-, -O-, -CH₂O- -NHC(O)NH-, -C(O)NH-, -aryl-(CE₂)_{z}-NHC(O)O-, -aryl-(CE₂)_{z}-NHC(0)NH-, or -CH₂CH₂NHC(O)-; wherein aryl is a divalent aryl; E is hydrogen or methyl; z is 0 or 1; p is an integer in the range from 0 to 30, r is 0 or 1, with the proviso that when p is 0, r is 0, and when p is in the range from 1 to 30, r is 1; (R⁸-O)ᵥ is a polyoxyalkylene, which is a homopolymer, a random copolymer or a block copolymer of C₂-C₄ oxyalkylene units, wherein R⁸ is C₂H₄, C₃H₆, C₄H₈, or a mixture thereof, v is an integer in the range from 5 to 250 preferably 5 to 100, more preferably 10 to 80, and most preferably 15 to 60; R⁹ is H or C₁-C₄ alkyl; and, D is a C₈-C₃₀ unsaturated alkyl, a carboxy-substituted C₈-C₃₀ unsaturated alkyl, or -R⁶CHC(R⁶)A-; wherein R⁶ is independently hydrogen, C₁-C₃₀ alkyl,-C(O)OH, or -C(O)OR⁷; R⁷ is C₁-C₃₀ alkyl; A is -CH₂C(O)O-, -C(O)O-, -O-, -CH₂O-, - NHC(O)NH-, -C(O)NH-, -aryl-(CE₂)_{z}-NHC(O)O-, -aryl-(CE₂)_{z}-NHC(O)NH-, or - CH₂CH₂NHC(O)-; aryl is a divalent aryl; E is hydrogen or methyl; z is 0 or 1. Preferably, the semi-hydrophobic surfactant unit is selected from having the following chemical formula:

CH₂=CH-O(CH₂)ₐO(C₃H₆₀)_{b}(C₂H₄₀)_{c}H,
or
CH₂=CHCH₂O(C₃H₆₀)_{d}(C₂H₄O)ₑH,

wherein a is 2, 3, or 4; b is an integer in the range of 1 to about 10; c is an integer in the range of about 5 to about 50; d is an integer in the range of 1 to about 10; and e is an integer in the range of about 5 to about 50.

Preferably, the first polymer comprises from about 0.1 to about 10 weight percent, which is of the total monomer mixture weight prior to polymerisation of the first polymer, of at least one semi-hydrophobic vinyl surfactant monomer.

An example of a suitable cationic polymer includes polyacrylate-1 crosspolymers, which are available under the trade name Carbopol^{®} Aqua CC Polymer from Noveon, Inc.

In a preferred embodiment, the first polymer further comprises at least one hydroxy-substituted non-ionic vinyl unit and/or at least one crosslinking unit. The hydroxy-substituted non-ionic vinyl unit is selected from a monomer being hydroxy-substituted (C₁-C₄)alkyl (meth)acrylate, such as 2-hydroxyethyl methacrylate, 2-hydroxyethyl acrylate, 3-hydroxypropyl acrylate, and the like; a hydroxy-substituted (C₁-C₄)alkyl (meth)acrylamide, such as N-(2-hydroxyethyl) methacrylamide, N-(2-hydroxyethyl) acrylamide, N-(3-hydroxypropyl) acrylamide, N-(2,3-dihydroxypropyl) acrylamide, allyl alcohol, glycerol monoallyl ether, 3-methyl-3-buten-1-ol, vinyl alcohol, vinyl acetate, or mixtures thereof.

Preferably, the first polymer comprises from about 0.01 to about 10 weight percent, which is of the total monomer mixture weight prior to polymerisation of the first polymer, of at least one hydroxy-substituted non-ionic vinyl monomer.

The crosslinking unit is selected from monomers being a (meth)acrylate esters of a polyols having at least two (meth)acrylate ester groups, such as trimethylolpropane triacrylate, trimethylolpropane dimethacrylate, triethylene glycol dimethacrylate, ethoxylated (30) bisphenol A dimethacrylate, or mixtures thereof.

Preferably, the first polymer comprises from about 0.01 to about 5 weight percent, which is of the total monomer mixture weight prior to polymerisation of the first polymer, of at least one crosslinking monomer.

The second polymer is selected from chitosan, chitosan salts, chitosan derivatives or mixtures thereof. Cellulose derivatives are also relevant, preferably cationic celluloses, more preferably those comprising at least one quaternary ammonium group, for example a copolymer of hydroxyethylcellulose and diallyldimethylammonium chloride, or the reaction product of hydroxyethylcellulose and an epoxide substituted by a trialkylammonium group. Preferably, the second polymer has a molecular weight from about 20000 g/mol to about 700000 g/mol. More preferably, the second polymer is selected from high and/or low molecular weight polymers,
wherein the low molecular weight is from 20000 to 100000 g/mol, and the high molecular weight is from 300000 to 700000 g/mol. Examples of suitable chitosan polymers include Derivat C XII from Kyowa Tecnos Co., Ltd, and Flonac N^{®} from Kyowa Tecnos Co., Ltd., respectively.

Preferably, the chitosan salt is chitosonium pyrrolidonecarboxylate, chitosonium lactide or chitosonium formate. Most preferably, the chitosan salt is chitosonium pyrrolidonecarboxylate, for example Kytamer^{®} PC from Amerchol Corporation.

Preferably, the chitosan derivatives are quaternised chitosan derivatives, alkylated chitosan derivatives or hydroxyalkylated chitosan derivatives, or mixtures thereof. The chitosan is 70 to 90% deacetylated, most preferably 75 to 80% deacetylated.

The composition may comprise a weight ratio of first polymer to second polymer of 15 : 0.5, preferably 10 : 0.25.

The composition comprises a cosmetically acceptable carrier. This carrier may be selected from either an aqueous medium or an aqueous-alcoholic medium. Preferably, the carrier is an aqueous medium.

When the carrier is an aqueous-alcoholic carrier, this carrier comprises water and an alcohol. Alcohols are usually incorporated into the hair styling compositions to improve the stability of the composition over time and to improve the hold factor confers to the hair-style by applying this composition onto hairs. The alcohol is preferably selected from alcohols having from one to five carbon atoms, more preferably from ethanol, isopropanol, n-propanol, ethylene glycol, glycerol, propylene glycols, or mixtures thereof, still more preferably from ethanol, isopropanol or mixtures thereof. This aqueous-alcoholic carrier comprises preferably from 0.01% to 10%, more preferably from 0.01% to 5%, still more preferably from 0.01% to 2%, alcohol, by weight of carrier. This carrier also comprises preferably from 90% to 99.9%, more preferably from 95% to 99.9%, still more preferably from 98% to 99.9%, water, by weight of carrier.

When the carrier is an aqueous carrier, this carrier consists essentially of water and is substantially free of alcohol. Preferably, the carrier is water. The inventors have surprisingly found that high-setting properties and a long-lasting hold are achieved with the composition, according to the present invention, even when the composition is substantially free of alcohol. The composition comprising both first and second polymers - as described above - and being substantially free of alcohol performs equally, or even better, than a composition comprising a significant proportion of alcohol. Without wishing to be bound by theory, it is believed that the properties of both the first and second polymers for conferring long-lasting hold and high-setting properties to a hair-style are independent to the presence or the absence of alcohols into the composition.

The composition comprises from 80% to 99%, preferably from 85% to 95%, more preferably from 87% to 92%, of a cosmetically acceptable carrier, by weight of the total composition.

The composition may further comprise a carboxylic acid. Preferably, the carboxylic acid is selected from glycolic acid, lactic acid or mixtures thereof. The composition may comprise from 0.01% to 5%, preferably from 0.05% to 2%, more preferably from 0.1% to 0.5%, of a carboxylic acid, by weight of the total composition. The inventors have surprisingly found that incorporating carboxylic acid into the cosmetic compositions, according to the present invention, enables the obtainment of compositions exhibiting even greater appealing characteristics.

In preferred embodiment, the present cosmetic composition is a hair care composition. More preferably, the cosmetic composition is a hair styling composition. Still more preferably, the cosmetic composition is a leave-on hair styling composition. In fact, the inventors have surprisingly found that there is no need for rinsing-off a composition comprising both polymers - as described above - as this composition leaves a minimum of residues, if any, on the hair after application and does not impart sticky, rough or unpleasant sensory feel to the hair or skin.

The present cosmetic composition may be in the form of a wax, mousse, lotion, emulsion, or gel. More preferably, the present cosmetic composition is in the form of a gel.

The viscosity of the composition is from about 20000 to about 40000 mPa·s measured at 25°C and a shear rate of 1 s⁻¹, spindle at CPE-52, using the device Brookfield DV-III Ultra Programmable Rheometer from Brookfield Engineering Laboratories.

The pH of the composition is preferably from about 3.5 to about 5.5. Most preferably the pH is from about 4 to about 4.5.

The composition may further comprise a surfactant. The composition preferably comprises from 0.3% to 2%, more preferably from 0.5% to 1.9%, still more preferably from 0.9% to 1.5%, of a surfactant, by weight of the total composition. When present, the surfactant is useful for emulsifying hydrophobic components, e.g. hydrophobic perfume oils, in order not to impair the transparency of the composition.

Suitable surfactants include any surfactant known to those skilled in the art as suitable for incorporation into a hair care composition. Suitable examples of surfactants may be found in the CTFA International Cosmetics Ingredient Dictionary and Handbook, "Surfactants", 10^{th} edition (2004). The surfactant may be selected from non-ionic surfactants, anionic surfactants, cationic surfactants, amphoteric surfactants, zwitterionic surfactants or mixtures thereof. The surfactant is selected preferably from ethoxylated castor oil, ethoxylated hydrogenated castor oil, or mixtures thereof. Castor oil and hydrogenated castor oil are preferably castor oils with a degree of ethoxylation from 10 to 100, more preferably from 20 to 60. The INCI name of these castor oils is usually PEG-x castor oil or PEG-x hydrogenated castor oil, wherein x is the degree of ethoxylation. Examples of commercially available ethoxylated hydrogenated castor oils include Cremophor CO 40^{®} (PEG-40 hydrogenated castor oil) and Cremophor CO 60^{®} (PEG-60 hydrogenated castor oil) from BASF Corporation.

The composition may further comprise an additional thickening polymer. As used herein, the expression "an additional thickening polymer" means a thickening polymer that is different from the first polymer as described above. The composition preferably comprises from 0.01% to 5%, more preferably from 0.1% to 3%, still more preferably from 0.2% to 2%, of a thickening polymer, by weight of the total composition.

Suitable thickening polymers include any thickening polymer known to those skilled in the art as suitable for incorporation into a hair care composition. Suitable examples of thickening polymers may be found in the CTFA International Cosmetics Ingredient Dictionary and Handbook, "Viscosity-increasing agents - Aqueous"; "Viscosity-increasing agents - Nonaqueous"; "Suspending agents - Nonsurfactants", 10th edition (2004). The thickening polymer may be selected from natural thickening polymers, synthetic thickening polymers or mixtures thereof. This thickening polymer is preferably soluble in the cosmetically acceptable carrier. A non-limiting list of natural thickening polymers includes guar gums, xanthan gums (e.g. Keltrol^{®} from CP Kelco), corn starch modified (e.g. Amaze^{®} from National Starch & Chemical Company), carrageenan gums (e.g. Viscarin GP209, NF^{®} from FMC Corporation), and hydroxyethylcellulose (e.g. Natrosol Hydroethylcellulose^{®} from Hercules Incorporated).

The composition may further comprise film formers. Film-forming polymers are understood as meaning those polymers which, when used in 0.01% to 5% strength aqueous, alcoholic or aqueous alcoholic or aqueous-alcoholic solution, are able to deposit a polymer film on the hair. An example of a suitable film former includes polyvinylpyrrolidone, such as Luviskol^{®} K30 to K120 from BASF Corporation.

The composition may further comprise an additional hair-fixing polymer. As used herein, the expression "an additional hair-fixing polymer" means a hair-fixing polymer that is different from the second polymer as described above. When present, the composition comprises preferably from 0.1% to 5%, more preferably from 0.1% to 2%, of an additional hair-fixing polymer, by weight of the total composition.

Preferably, the additional hair-fixing polymer is selected from anionic hair-fixing polymer, amphoretic hair-fixing polymer, cationic hair-fixing polymer, or mixtures thereof. Suitable additional hair-fixing polymers may be found in the CTFA International Cosmetics Ingredient Dictionary and Handbook, "Hair fixatives", 10th edition (2004). The additional cationic hair-fixing polymer may be selected from polyquaternium-6; polyquaternium-7; polyquaternium-10; polyquaternium-11; polyquaternium-16; polyquaternium-28; polyquaternium-35; polyquaternium-37; polyquaternium-47; polyquaternium-57; polyquaternium-80; copolymers of polyvinylpyrrolidone and imidazolimine methochloride; terpolymer of dimethyldiallyl ammonium chloride, sodium acrylate and acrylamide; terpolymer of vinylpyrrolidone, dimethyl aminoethyl methacrylate and vinyl caprolactam; quaternary ammonium salt of hydroxyethyl cellulose and a trimethylammonium substituted epoxide; or mixtures thereof. The amphoretic hair-fixing polymer may be selected from copolymers of octoylacrylamide, t-butylamino-ethylmethacrylate and two or more monomers, comprising acrylic acid, methacrylic acid or their esters; isobutylene/ethylmaleimide/hydroxyethylmaleimide copolymer; or mixtures thereof.

The composition may further comprise particles. When present, the composition preferably comprises from 0.005% to 5%, more preferably from 0.01% to 3%, particles, by weight of the total composition. The particles may be selected from particles comprising at least one polyolefin, silica particles, or mixtures thereof. The particles comprising at least one polyolefin preferably comprise polyethylene, polypropylene or mixtures thereof. An example of commercially available particles comprising at least one mica, titanium oxide or iron oxide includes Flonac^{®} M from Eckart GmbH & Co. KG.

A variety of additional optional ingredients may be incorporated into the composition. Non-limiting examples of these additional ingredients include fragrances and perfume oils; preservatives, such as parabene, iodopropynyl butylcarbamate, or mixtures thereof; moisturiser; opacifying agents, such as ethylenglycol distearate, fatty acid monoalkylolamide, or mixtures thereof; buffer substances, such as sodium citrate, sodium phosphate, or mixtures thereof; hair care substances, such as betaine, panthenol, plant extracts, vegetable extracts, protein hydroylsates, silk hydrolysates, lanolin derivatives, or mixtures thereof; physiologically compatible silicone derivatives, such as volatile or non-volatile silicone oils, high molecular weight siloxane polymers or mixtures thereof; light protective agents; antioxidants; radical-trapping agents; anti-dandruff agents; fatty alcohols; vitamins; direct dye compounds; and, lustre-imparting substances; and, pigments.

According to the second aspect, the present invention relates to a method of treating hair and/or skin comprising the steps of:
(a) providing a composition,
(b) applying said composition onto hair and/or the scalp; and,
(c) achieving a hair style.

The composition may be applied as a hand gel or as a spray gel. When applied as a hand gel, the method may comprise the steps of dispensing this composition into the palm of one hand, rubbing both hands altogether to spread this composition onto palms, and then rubbing and/or massaging the hairs with the palms to apply this composition evenly onto hairs. When applied as a spray gel, the method may comprise the steps of actuating the dispenser pump for dispensing the composition as a spray and, simultaneously, directing the spray to allow an even application of the composition onto hairs.

It is preferably applied from 15 to 45 mg (per gram of hair), more preferably from 20 to 40 mg (per gram of hair), still more preferably from 25 to 35 mg (per gram of hair), of the composition onto hairs. This is advantageous for achieving a hair style having long-lasting hold with high-setting properties.

The composition is preferably applied onto hair as a spray. The composition may be applied using a hair styling kit, according to the second aspect of the invention. The spraying of the composition onto hairs is advantageous as it allows an easy and even distribution onto hairs of the composition.

The hair style may be achieved by working hairs with fingers till the desired hair style is obtained.

The method may further comprise a step of applying an aqueous medium onto hairs, before applying the hair styling composition onto hairs. The aqueous medium is preferably water, more preferably tap water. This medium may be applied onto hairs by wetting hands and then massaging evenly hairs with the hands. The medium may also be applied onto hairs by spraying this medium onto hairs, e.g. by using a spray dispenser. The medium may also be applied by wetting the hairs with tap water, then shampooing the hairs with a conventional shampoo, then rinsing-off the hairs with water and removing the water in excess by massaging the hairs with a towel. The application of an aqueous medium before applying the composition is advantageous as the application of the hair styling composition is facilitated, allowing an even distribution, all over the hairs of the composition. The application of an aqueous medium before applying the composition is also advantageous as it increases the adhesion the first and second quaternary ammonium salts onto hairs, and so it increases the degree of setting of the hair style.

The method may further comprise the step of blow-drying hairs. Hairs may be blow-dried by using a conventional blow-drier. This step is preferably performed simultaneously to the step of achieving the hair style.

This method is particularly suitable for achieving a hair-style having long-lasting hold with high-setting properties. A hair style with high-setting properties has preferably a 3 point bending force from 0.1 N to 10 N, preferably from 1.8 N to 6 N.

The hair style having long-lasting hold has preferably a hold factor from 30% to 100%, preferably from 70%, to 100% after 24 hours. The provision of a composition having a hold factor from 30% to 100% after 24 hours, is advantageous in order to achieve a sufficient hold over a period of time, preferably for at least 5 hours, and more preferably for at least 24 hours.

According to the third aspect, the present invention relates to a use of the composition for achieving a hair style having at least one of curl definition, shine, hold and/or volume.

### Examples

The following examples further describe and demonstrate the preferred embodiments within the scope of the present invention. The examples are given solely for the purpose of illustration, and are not to be construed as limitations of the present invention since many variations thereof are possible without departing from its scope.

All weights provided in these examples are weights of the commercially available materials, including active(s) and/or solvent and/or by-products.

The compositions in the form of a gel are prepared as follows in table 1:

**Table 1**

| Ingredients (%w/w) | Example 1 | Example 2 | Example 3 |
|---|---|---|---|
| Water¹ | QSP | QSP | QSP |
| Polyacrylate-1 crosspolymer² | 10.000 | 10.000 | 10.000 |
| Lactic acid 90% | 0.730 | 1.050 | - |
| Glycolic acid | - | - | 0.670 |
| High molecular weight chitosan³ | 0.250 | - | 0.250 |
| Low molecular weight chitosan⁴ | - | 0.300 | - |
| Poly-vinyl-pyrrolidone⁵ 20% | 10.000 | 10.000 | 10.000 |
| Benzyl alcohol | 0.500 | 0.500 | - |
| DMDM hydantoin⁶ | 0.500 | 0.500 | 0.500 |
| Fragrance | 0.200 | 0.200 | 0.200 |
| Pantothenyl ethyl ether | 0.090 | 0.090 | 0.090 |
| DL-Panthenol 50% | 0.020 | 0.020 | 0.020 |
| PEG-40 Hydrogenated castor oil | 0.400 | 0.400 | 0.400 |
| Phenoxyethanol + ethylhexylglycerin⁷ | - | - | 0.500 |
| Glycerin 86% | - | - | 0.500 |

| | | | |
|---|---|---|---|
| 1: Deionised water; 2: Carbopol^{®} Aqua CC from Noveon Inc.; 3: Flonac N^{®} from Kyowa Tecnos Co., Ltd.; 4: Derivat C XII from Kyowa Tecnos Co., Ltd.; 5: Luviskol^{®} K85 from BASF Corporation; 6: Nipaguard^{®} DMDMH from Clairant International Ltd.; 7: Euxyl PE 9010 from Schulke & Mary GmbH. | | | |

The compositions of examples 1 to 3 exhibit long-lasting hold, high hair-setting properties and satisfactory consistency/rheology altogether with appealing characteristics

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A cosmetic composition comprising:
(a) a first polymer comprising at least one amino-substituted vinyl unit, at least one hydrophobic non-ionic vinyl unit, at least one associative vinyl unit, and at least one semi-hydrophobic vinyl surfactant unit;
(b) a second polymer selected from chitosan, chitosan salts, chitosan derivatives or mixtures thereof;
(c) a cosmetically acceptable carrier.

2. A composition, according to claim 1, wherein the first polymer further comprises at least one hydroxy-substituted non-ionic vinyl unit and/or at least one crosslinking unit.

3. A composition, according to any of the preceding claims, wherein the amino-substituted vinyl unit is selected from monomers being a mono-(C₁-C₄)alkylamino(C₁-C₈)alkyl (meth)acrylate; di-(C₁-C₄)alkylamino(C₁-C₈)alkyl (meth)acrylate; mono-(C₁-C₄)alkylamino(C₁-C₈)alkyl (meth)acrylamide; di-(C₁-C₄)alkylamino(C₁-C₈)alkyl (meth)acrylamide; nitrogen-containing heterocyclic (meth)acrylamide; nitrogen-containing heterocyclic (meth)acrylate; or mixtures thereof.

4. A composition, according to any of the preceding claims, wherein the hydrophobic non-ionic vinyl unit is selected from monomers being a C₁-C₃₀ alkyl (meth)acrylate; C₁-C₃₀ alkyl (meth)acrylamide; styrene; substituted styrene; vinyl ester; unsaturated nitrile; unsaturated silane; and mixtures thereof.

5. A composition, according to any of the preceding claims, wherein the associative vinyl unit is selected from monomers having the formula (I): 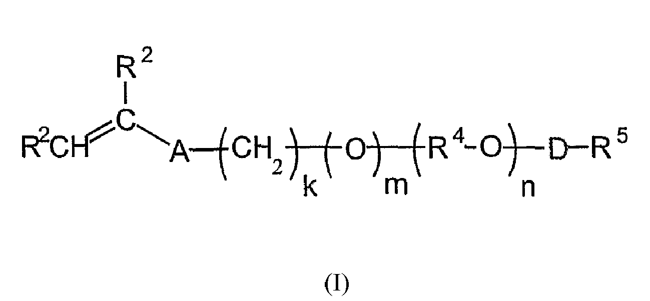 wherein R² is independently H, methyl, -C(O)OH, or -C(O)OR³; with R³ being C₁-C₃₀ alkyl;
A is -CH₂C(O)O-, -C(O)O-, -O-, -CH₂O- -NHC(O)NH-, -C(O)NH-, -aryl-(CE₂)₂-NHC(O)O-, -aryl-(CE₂)_{z}-NHC(0)NH-, or -CH₂CH₂NHC(O)-; wherein aryl is a divalent aryl; E is hydrogen or methyl; z is 0 or 1;
k is an integer in the range of 0 to 30;
m is an integer being 0 or 1, with the proviso that when k is 0, m is 0, and when k is in the range of 1 to about 30, m is 1;
(R⁴-O)ₙ is a polyoxyalkylene, which is a homopolymer, a random copolymer, or a block copolymer of C₂-C₄ oxyalkylene units, wherein R⁴ is C₂H₄, C₃H₆, C₄H₈, or a mixture thereof, and n is an integer in the range of 5 to 250;
D is -R⁴O-, -R⁴NH-, -C(O)-, -C(O)NH-, -R⁴NHC(O)NH-, or -C(O)NHC(O)-; and,
R⁵ is a substituted or unsubstituted alkyl selected from the group consisting of a C₈-C₄₀ linear alkyl, a C₈-C₄₀ branched alkyl, a C₈-C₄₀ carbocyclic alkyl, a C₂-C₄₀ alkyl-substituted phenyl, an aryl-substituted C₂-C₄₀ alkyl, and a C₈-C₈₀ complex ester; wherein the R⁵ alkyl group optionally comprises one or more substituents selected from the group consisting of a hydroxyl group, an alkoxyl group, and a halogen group.

6. A composition, according to any of the preceding claims, wherein the semi-hydrophobic vinyl surfactant unit has the formula (II): 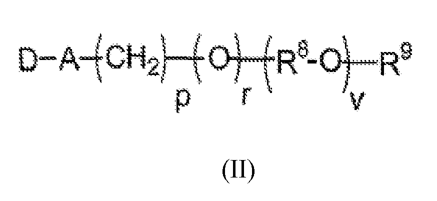 wherein A is -CH₂C(O)O-, -C(O)O-, -O-, -CH₂O-, -NHC(O)NH-, -C(O)NH-, -aryl-(CE₂)_{z}-NHC(O)O-, -aryl-(CE₂)_{z}-NHC(O)NH-, or -CH₂CH₂NHC(O)-; wherein aryl is a divalent aryl; E is hydrogen or methyl; z is 0 or 1;
p is an integer in the range from 0 to 30, r is 0 or 1, with the proviso that when p is 0, r is 0, and when p is in the range from 1 to 30, r is 1;
(R⁸-O)ᵥ is a polyoxyalkylene, which is a homopolymer, a random copolymer or a block copolymer of C₂-C₄ oxyalkylene units, wherein R⁸ is C₂H₄, C₃H₆, C₄H₈, or a mixture thereof,
v is an integer in the range from 5 to 250;
R⁹ is H or C₁-C₄ alkyl; and,
D is a C₈-C₃₀ unsaturated alkyl, a carboxy-substituted C₈-C₃₀ unsaturated alkyl, or - R⁶CHC(R⁶)A-; wherein R⁶ is independently hydrogen, C₁-C₃₀ alkyl,-C(O)OH, or - C(O)OR⁷; R⁷ is C₁-C₃₀ alkyl; A is -CH₂C(O)O-, -C(O)O-, -O-, -CH₂O-, -NHC(O)NH-, - C(O)NH-, -aryl-(CE₂)_{z}-NHC(O)O-, -aryl-(CE₂)_{z}-NHC(O)NH-, or -CH₂CH₂NHC(O)-; aryl is a divalent aryl; E is hydrogen or methyl; z is 0 or 1.

7. A composition, according to any of the preceding claims, wherein the hydroxy-substituted non-ionic vinyl unit is selected from a monomer being a hydroxy-substituted (C₁-C₄)alkyl (meth)acrylate; hydroxy-substituted (C₁-C₄)alkyl (meth)acrylamide, allyl alcohol, glycerol monoallyl ether, 3-methyl-3-buten-1-ol, vinyl alcohol, vinyl acetate, or mixtures thereof.

8. A composition, according to any of the preceding claims, wherein the crosslinking unit is selected from monomers being a (meth)acrylate ester of a polyol having at least two (meth)acrylate ester groups.

9. A composition, according to any of the preceding claims, wherein the chitosan has a molecular weight from 20000 g/mol to 700000 g/mol.

10. A composition, according to any of the preceding claims, wherein the chitosan derivatives are selected from quaternised chitosan derivatives, alkylated chitosan derivatives, hydroxyalkylated chitosan derivatives, or mixtures thereof.

11. A composition, according to any of the preceding claims, wherein this composition is a hair care composition, preferably a hair styling composition.

12. A composition, according to any of the preceding claims, wherein this composition is a leave-on composition.

13. A composition, according to any of the preceding claims, wherein this composition is in the form of a gel.

14. A composition, according to any of the preceding claims, further comprising a carboxylic acid, preferably wherein the carboxylic acid is selected from glycolic acid, lactic acid or mixtures thereof.

15. A composition, according to any of the preceding claims, wherein the viscosity is from 20000 to 40000 mPa·s measured at 25°C and a shear rate of 1 s⁻¹.

16. Method of treating hair comprising the steps of:
(a) providing a composition, as defined in any of claims 1 to 15,
(b) applying said composition onto hair and/or the scalp; and,
(c) achieving a hair style.

17. Use of the composition, as defined in any of claims 1 to 15, for achieving a hair style having at least one of curl definition, shine, hold and/or volume.
